(19) Europäisches Patentamt
European Patent Office
Office européen des brevets

(11) Numéro de publication: **0 378 937 A1**

(12) **DEMANDE DE BREVET EUROPEEN**

(21) Numéro de dépôt: **89403489.1**

(51) Int. Cl.[5]: **A61K 7/13**

(22) Date de dépôt: **14.12.89**

Revendications pour les Etats contractants suivants: ES

(30) Priorité: **16.12.88 FR 8816686**

(43) Date de publication de la demande: **25.07.90 Bulletin 90/30**

(84) Etats contractants désignés: **AT BE CH DE ES FR GB GR IT LI NL SE**

(71) Demandeur: **L'OREAL**
**14, Rue Royale**
**F-75008 Paris(FR)**

(72) Inventeur: **Bore, Pierre**
**197 avenue Daniel Perdrigé**
**F-93370 Montfermeil(FR)**
Inventeur: **De Labbey, Arnaud**
**9, rue Charles Dordain**
**F-93600 Aulnay-sous-Bois(FR)**
Inventeur: **Baudry, Alain**
**19 Villa du Buisson Ardent**
**F-95500 Gonesse(FR)**

(74) Mandataire: **Casalonga, Axel et al**
**BUREAU D.A. CASALONGA - JOSSE**
**Morassistrasse 8**
**D-8000 München 5(DE)**

(54) **Procédé de teinture des fibres kératiniques avec des dérivés d'indole associés à des nitrites et composition de mise en oeuvre.**

(57) L'invention est relative à un procédé de teinture des fibres kératiniques, comprenant l'application d'une composition (A) contenant un dérivé d'indole répondant à la formule (I) :

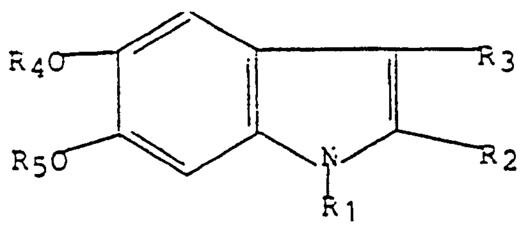

(I)

dans laquelle $R_1$ représente d'hydrogène, alkyle ou $-SiR_9R_{10}R_{11}$ ;
$R_2$ et $R_3$ représentent d'hydrogène, alkyle, carboxyle, alcoxycarbonyle ou $-COOSiR_9R_{10}R_{11}$ ;
$R_4$ et $R_5$, représentent hydrogène, alkyle, formyle, acyle, alcénoyle, $-SiR_9R_{10}R_{11}$, $-P(O)(OR_6)_2$, $R_6OSO_2-$, ou bien $R_4$ et $R_5$, conjointement avec les atomes d'oxygène auxquels ils sont rattachés, forment un cycle ; $R_6$ et $R_7$ représentant hydrogène ou alkyle, $R_8$ représentant un alcoxy ou mono ou dialkylamino, $R_9$, $R_{10}$ et $R_{11}$ représentant alkyle, l'un au moins des radicaux $R_1$ à $R_5$ étant différent d'hydrogène, et les sels, l'application de la composition (A) étant suivie par l'application de la composition (B), constituée par une composition aqueuse présentant un pH acide, la composition (A) ou (B) contenant au moins un nitrite.

EP 0 378 937 A1

# Procédé de teinture des fibres kératiniques avec des dérivés d'indole associés à des nitrites et composition de mise en oeuvre.

La présente invention est relative à un nouveau procédé de teinture des fibres kératiniques et en particulier des cheveux humains, mettant en oeuvre des dérivés d'indole associés à des nitrites et aux compositions mises en oeuvre dans ce procédé.

Il est bien connu que la biosynthèse naturelle des eumélanines à partir de la tyrosine se fait en plusieurs étapes. L'une d'elles consiste en la formation du 5,6-dihydroxyindole qui s'oxyde pour former un pigment qui est l'un des constituants principaux de l'eumélanine.

On a déjà proposé dans le passé différents procédés de teinture des fibres kératiniques et en particulier des cheveux humains, mettant en oeuvre le 5,6-dihydroxyindole ou des dérivés d'indole.

On peut ainsi citer les brevets français de la demanderesse n° 1.133.594, 1.166.172, 2.390.158, la demande de brevet français 2.536.993, ainsi que les demandes de brevets français n° 2.594.331, 2.593.061, 2.606.636, de la demanderesse.

Certains de ces procédés mettent en oeuvre des cations métalliques qui posent un certain nombre de problèmes, dans la mesure où l'élimination de ces cations métalliques du cheveu est relativement difficile. Après deux traitements et malgré les rinçages, il reste souvent des traces de métaux sur les cheveux qui peuvent présenter des inconvénients lorsque les cheveux doivent subir des traitements ultérieurs comme des décolorations ou des permanentes. C'est en particulier le cas du cuivre et du manganèse.

D'autres procédés mettent en oeuvre des ions iodure. Ces procédés comportent cependant également l'utilisation d'eau oxygénée, ce qui présente l'inconvénient d'être dégradant pour le cheveu.

La demanderesse a découvert maintenant qu'il était possible d'effectuer une teinture à l'aide de dérivés indoliques sans utiliser de peroxyde d'hydrogène, ni d'ammoniaque, ni un cation métallique, en imprégnant dans un premier temps les fibres kératiniques et en particulier les cheveux par les dérivés indoliques, et dans un deuxième temps par une composition acide, l'une au moins de ces compositions contenant un nitrite.

La demanderesse a découvert qu'il était facile de teindre les cheveux naturels dans des tons clairs ou foncés.

L'invention a donc pour objet un nouveau procédé de teinture mettant en oeuvre au moins un dérivé d'indole et un nitrite.

Un autre objet de l'invention est constitué par les compositions et le dispositif à plusieurs compartiments utilisés dans le cadre de ce procédé.

D'autres objets de l'invention apparaîtront à la lecture de la description et des exemples qui suivent.

Le procédé de teinture des fibres kératiniques et en particulier des cheveux, conforme à l'invention, est essentiellement caractérisé par le fait que l'on applique sur ces fibres au moins une composition (A) contenant, dans un milieu approprié pour la teinture, au moins un dérivé d'indole, répondant à la formule :

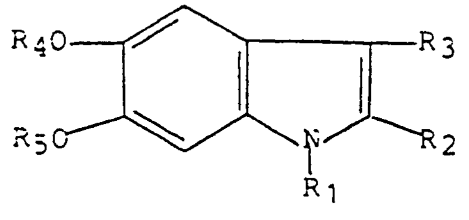

(I)

dans laquelle $R_1$ représente un atome d'hydrogène, un groupement alkyle inférieur ou un groupement $-SiR_9R_{10}R_{11}$ ;

$R_2$ et $R_3$, identiques ou différents, représentent un atome d'hydrogène ou bien un groupement alkyle inférieur, un groupement carboxyle, un groupement alcoxycarbonyle inférieur ou un groupement $-COOSiR^9R^{10}R^{11}$;

$R_4$ et $R_5$, identiques ou différents, représentent un atome d'hydrogène, un groupement alkyle linéaire ou ramifié en $C_1$-$C_{20}$, un groupement formyle, un groupement acyle en $C_2$-$C_{20}$, linéaire ou ramifié, un groupement alcénoyle en $C_3$-$C_{20}$, linéaire ou ramifié, un groupement $-SiR_9R_{10}R_{11}$, un groupement $-P(O)-(OR_6)_2$, un groupement $R_6OSO_2$-, ou bien $R_4$ et $R_5$, conjointement avec les atomes d'oxygène auxquels ils sont rattachés, forment un cycle contenant éventuellement un groupement carbonyle, un groupement méthylène, un groupement thiocarbonyle ou un groupement :

> P(O)OR$_6$, ou bien > CR$_7$R$_8$ ;

R$_6$ et R$_7$ représentant un atome d'hydrogène ou un groupement alkyle inférieur, R$_8$ représentant un groupement alcoxy inférieur ou un groupement mono ou dialkylamino, R$_9$, R$_{10}$ et R$_{11}$, identiques ou différents, représentant des groupements alkyle inférieurs, linéaires ou ramifiés, l'un au moins des radicaux R$_1$ à R$_5$ étant différent d'hydrogène, et les sels d'addition avec des acides minéraux ou organiques, comme par exemple les chlorhydrates et bromhydrates, ainsi que les sels de métaux alcalins, alcalino-terreux ou d'amines correspondants,

l'application de la composition (A) étant suivie par l'application d'une composition aqueuse acide (B), la composition (A) ou la composition (B) contenant au moins un nitrite.

Pour les composés de formule (I) définis ci-dessus, le groupement alkyle inférieur ou alcoxy inférieur désigne de préférence un groupement ayant 1 à 6 atomes de carbone.

Parmi les composés de formule (I), les composés particulièrement préférés sont ceux répondant à la formule :

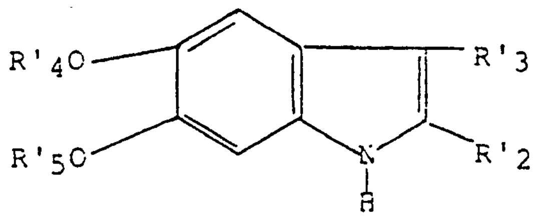

(II)

dans laquelle R'$_2$ et R'$_3$, identiques ou différents, représentent un atome d'hydrogène, un groupement alkyle inférieur en C$_1$-C$_6$, un groupement carboxyle, un groupement alcoxycarbonyle inférieur en C$_1$-C$_6$ pour le groupement alcoxy, R'$_4$ et R'$_5$, identiques ou différents, représentent un atome d'hydrogène, un groupement alkyle inférieur en C$_1$-C$_6$, un groupement acyle linéaire en C$_2$-C$_{20}$, et de préférence en C$_2$-C$_{14}$, un groupement triméthylsilyle, ou bien R'$_4$ et R'$_5$ conjointement avec les atomes d'oxygène auxquels ils sont rattachés, forment un cycle méthylènedioxy ou carbonyldioxy, l'un au moins des radicaux R'$_4$ ou R'$_5$ étant différent d'hydrogène.

Parmi les composés de formule (I) plus particulièrement préférés, on peut citer le 5-méthoxy 6-hydroxyindole, le 1-méthyl, 5,6-dihydroxyindole, le 3-méthyl 5,6-dihydroxyindole, le 2-méthyl 5,6-dihydroxyindole, le 2,3-diméthyl 5,6-dihydroxyindole, le 5,6-di(triméthylsilyloxy)indole, le 5-(ou 6-)myristoyloxy 6-(ou 5-)hydroxyindole, le 5,6-(méthylènedioxy)indole, le 5-hydroxy 6-acétoxyindole, le 5-acétoxy 6-hydroxyindole, le 2-éthoxycarbonyl 5,6-dihydroxyindole, le 5-hydroxy 6-méthoxyindole, le 5,6-carbonyldioxyindole et le 2-carboxy 5,6-dihydroxyindole et leurs sels.

Les nitrites plus particulièrement utilisables, conformément à l'invention, sont :

- des nitrites de métaux alcalins, alcalino-terreux ou d'ammonium ou de tout autre cation cosmétiquement acceptable lorsqu'il est utilisé pour la teinture des cheveux humains vivants ;
- des dérivés organiques de nitrite, tels que par exemple le nitrite d'amyle ;
- ou encore des vecteurs de nitrite, c'est-à-dire des composés qui, par transformation, génèrent un nitrite.

Les nitrites particulièrement préférés sont les nitrites de sodium, de potassium ou d'ammonium.

Les dérivés d'indole sont utilisés dans la composition (A) dans des quantités suffisantes pour teindre, après association avec le nitrite, les fibres kératiniques suivant la nuance désirée. Cette concentration est comprise de préférence entre 0,01 et 0,3 mole/litre.

L'anion nitrite exprimé sous forme de $NO_2^-$ est présent dans des quantités suffisantes pour développer avec les dérivés d'indole de formules (I) ou (II) définies ci-dessus, une coloration. Sa concentration est de préférence compris entre 0,02 et 1 mole/litre.

Le pH de la composition (A) est de préférence compris entre 2 et 10. Le pH de la composition (B) est acide et permet de réguler la coloration.

En milieu acide, le nitrite est suffisamment oxydant pour réagir avec les dérivés d'indole de formule (I) qui peuvent ensuite, par polymérisation, conduire à la formation du pigment.

En milieu alcalin, le nitrite n'est plus assez oxydant pour oxyder les dérivés d'indole de formule (I).

Le pH de la composition (B), conformément à l'invention, est compris entre 2 et 6 et de préférence est ajusté à environ 3 pour des durées de contact faibles et pour les nuances les plus foncées.

Une forme de réalisation préférée de l'invention consiste à appliquer dans un premier temps, la composition (A) contenant le dérivé d'indole de formule (I) ou (II) et dans un second temps, la composition (B) contenant en milieu aqueux et acide un nitrite.

Une autre forme de réalisation de l'invention consiste à appliquer dans un premier temps, une

composition (A') contenant dans un milieu approprié pour la teinture, la dérivé d'indole de formule (I) ou (II) en milieu neutre ou alcalin et un nitrite. Dans ce cas, on teint les cheveux en appliquant dans un deuxième temps, une composition contenant un milieu aqueux approprié pour la teinture, ajusté à un pH acide et de préférence compris entre 2 et 6.

Un autre objet de l'invention est donc constitué par une telle composition tinctoriale (A') contenant simultanément le dérivé d'indole de formule (I) ou (II) et le nitrite.

Les proportions en dérivés d'indole et en nitrites ainsi que la nature des nitrites, sont celles indiquées ci-dessus.

Les compositions (A) ou (B) ainsi que la composition (A') contenant le dérivé d'indole de formule (I) ou (II) et de nitrite peuvent contenir des adjuvants habituellement utilisés en teinture des fibres kératiniques et plus particulièrement, en plus de l'eau, un solvant qui, dans le cas où la composition est appliquée sur les cheveux, est cosmétiquement acceptable.

Les solvants sont choisis parmi les solvants organiques, tels que les alcools inférieurs en $C_1$-$C_6$ comme l'alcool éthylique, l'alcool propylique ou isopropylique, l'alcool tertiobutylique, l'éthylène glycol, le propylèneglycol, les éthers monométhylique, monoéthylique ou monobutylique de l'éthylèneglycol, l'acétate du monoéthyléther de l'éthylèneglycol, les monométhyléthers du propylèneglycol et du dipropylène glycol, le lactate de méthyle.

Les solvants particulièrement préférés sont l'alcool éthylique et le propylèneglycol.

Les solvants sont utilisés plus particulièrement dans des concentrations comprises entre 10 et 50% pour les alcools inférieurs lorsque la concentration en dérivés d'indole de formule (I) ou (II) dépasse 1% en poids par rapport au poids total de la composition.

Les compositions utilisables dans le procédé conforme à l'invention peuvent également contenir des agents tensio-actifs anioniques, cationiques, non ioniques, amphotères ou leurs mélanges, présents de préférence dans des proportions comprises entre 0,1 et 50%, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

Les épaississants peuvent être choisis plus particulièrement parmi l'alginate de sodium, la gomme arabique, la gomme de guar, les biopolymères comme la gomme de xanthane ou les scléroglucanes, les dérivés de cellulose tels que la méthylcellulose, l'hydroxyéthyl cellulose, l'hydroxypropylméthylcellulose, le sel de sodium de la carboxyméthylcellulose et les polymères d'acide acrylique. On peut également utiliser des agents épaississants minéraux tels que la bentonite.

Ces épaississants, utilisés seuls ou en mélange, sont présents de préférence dans des proportions comprises entre 0,1 et 5% en poids par rapport au poids total de la composition et avantageusement entre 0,5 et 3% en poids.

Les agents d'acidification utilisables sont plus particulièrement choisis parmi l'acide lactique, l'acide acétique, l'acide tartrique, l'acide phosphorique, l'acide chlorhydrique, l'acide citrique.

Les agents alcalinisants sont plus particulièrement choisis parmi les amines, telles que les alcanolamines, les alkylamines ou alors les hydroxydes ou les carbonates alcalins ou d'ammonium.

Les compositions (A), (A') et/ou (B) utilisables dans le procédé conforme à l'invention, peuvent contenir d'autres colorants habituellement utilisés pour la teinture des fibres kératiniques et en particulier des colorants directs tels que les dérivés nitrés benzéniques, les colorants d'oxydation tels que que les précurseurs de colorants d'oxydation de type para ou ortho, des coupleurs ou des colorants d'oxydation dits "rapides", c'est-à-dire des molécules à structure benzénique, précurseurs de colorants, susceptibles de générer les composés colorés par simple oxydation à l'air pendant le temps de pose sur la chevelure, généralement inférieur à 1 heure et en l'absence de tout autre agent oxydant.

Ces compositions peuvent également contenir le 5,6-dihydroxyindole.

Ces compositions peuvent être formulées sous des formes diverses, cosmétiquement acceptables, lorsqu'elles sont appliquées sur les cheveux, telles que de liquide plus ou moins épaissis ou gélifiés, de crèmes, d'émulsions, de mousses ou d'autres formes appropriées pour réaliser la teinture.

En vue de la mise en oeuvre du procédé conforme à l'invention, on peut conditionner les différentes composition dans un dispositif à plusieurs compartiments encore appelé "kit" ou nécessaire de teinture, comportant tous les composants destinés à être appliqués pour une même teinture sur les fibres kératiniques et en particulier les cheveux, en application successive, avec ou sans prémélange.

De tels dispositifs sont connus en eux-mêmes et peuvent comporter un premier compartiment renfermant la composition (A) contenant les dérivés d'indole de formule (I) ou (II) dans un milieu approprié pour la teinture, dans un deuxième compartiment, la composition (B) contenant dans un milieu approprié pour la teinture, à un pH acide, un nitrite tel que défini ci-dessus.

Une autre forme de réalisation consiste à prévoir un dispositif à plusieurs compartiments ou "kit" comportant dans un premier compartiment la composition (A′) contenant dans un milieu approprié pour la teinture et présentant un pH neutre ou alcalin, les dérivés d'indole de formule (I) ou (II) et un nitrite et dans un second compartiment, une solution aqueuse présentant un pH acide compris de préférence entre 2 et 6.

Le procédé conforme à l'invention ainsi que les compositions définies ci-dessus peuvent être mis en oeuvre pour teindre les cheveux naturels ou déjà teints, permanentés ou non ou défrisés, ou des cheveux fortement ou légèrement décolorés, éventuellement permanentés. Dans ce cas, on applique la composition (A) à une température qui est celle de la tête, c'est-à-dire comprise entre 25 et 35 °C, pendant 5 à 30 minutes et on fait suivre avec ou sans rinçage intermédiaire, cette application par l'application de la composition (B) contenant le nitrite qui est maintenu au contact des cheveux pendant 5 à 30 minutes. La température de la teinture est également celle de la tête et elle est comprise entre 25 et 35 °C.

Dans la forme de réalisation mettant en oeuvre les dérivés d'indole de formule (I) ou (II) et le nitrite dans une même composition en milieu neutre ou alcalin, on maintient la première composition (A′) au contact des cheveux pendant 5 à 30 minutes et après un rinçage éventuel, on applique la composition aqueuse acide.

Il est également possible d'utiliser ces compositions pour la teinture des fourrures ou de la laine.

Les exemples suivants sont destinés à illustrer l'invention sans pour autant présenter un caractère limitatif.

## EXEMPLE 1

On imprègne une mèche de 1 g de cheveux naturels gris à 90% de blancs par 5 g d'une composition (A) suivante :

| Composition (A) | |
|---|---|
| - 5-méthoxy 6-hydroxyindole | 2,5 g |
| - Ethanol | 40,0 g |
| - Acide polyacrylique réticulé, vendu par la Société GOODRICH sous là dénomination CARBOPOL 940 | 3,5 g |
| - Eau | qsp 100,0 g |
| Le pH spontané est d'environ 3,5 | |

La composition est maintenue au contact des cheveux pendant 15 minutes, suivi d'un rinçage à l'eau courante et d'un essorage.

On applique ensuite sur la mèche 5 g de la composition (B) suivante :

| Composition (B) | |
|---|---|
| - Nitrite de sodium | 2,5 g |
| - Solution aqueuse à 20% d'acide polyacrylamide méthylpropanesulfonique | 25,0 g |
| - Acide chlorhydrique qs pH = 3 | |
| - Eau | qsp 100,0 g |

Cette composition est maintenue au contact des cheveux pendant 10 minutes.

On rince ensuite la mèche, on lave avec un shampooing à 5% de laurylsulfate de sodium. On obtient de cette façon une mèche dont la couleur est teinte en châtain foncé.

## EXEMPLE 2

On imprègne une mèche de 1 g de cheveux naturels gris à 90% de blancs par 5 g d'une composition

(A) suivante :

| Composition (A) | |
|---|---|
| - 5-hydroxy 6-méthoxyindole<br>- Ethanol<br>- Eau | 2,5 g<br>30,0 g<br>qsp 100,0 g |
| Le pH spontané est de 5,6 | |

La composition (A) est maintenue au contact des cheveux pendant 15 minutes. On rince ensuite la mèche. On l'essore. On imprègne ensuite la mèche par 5 g de la composition (B) définie dans l'exemple 1.

On maintient cette composition au contact des cheveux pendant 9 minutes, puis on rince.

On lave au shampooing avec une solution de laurylsulfate de sodium à 5%. On rince à nouveau et on sèche. On obtient une coloration châtain très foncé.

## EXEMPLE 3

On imprègne une mèche de 1 g de cheveux naturels gris à 90% de blancs par 5 g d'une composition (A) suivante :

| Composition (A) | |
|---|---|
| - 1-méthyl 5,6-dihydroxyindole<br>- Ethanol<br>- Eau | 2,5 g<br>40,0 g<br>qsp 100,0 g |
| Le pH spontané est d'environ 5,5. | |

La composition est maintenue au contact des cheveux pendant 15 minutes, suivi d'un rinçage à l'eau courante et d'un essorage.

On applique ensuite sur la mèche 5 ml de la composition (B) définie dans l'exemple 1.

Cette composition est maintenue au contact des cheveux pendant 9 minutes. On rince ensuite la mèche. On lave avec un shampooing à 5% de laurylsulfate de sodium. On obtient de cette façon une mèche dont la couleur est châtain à reflets jaunâtres.

## EXEMPLE 4

On imprègne une mèche de 1 g de cheveux naturels gris à 90% de blancs par 5 g d'une composition (A) suivante :

| Composition (A) | |
|---|---|
| - 3-méthyl 5,6-dihydroxyindole<br>- Ethanol<br>- Eau | 2,5 g<br>40,0 g<br>qsp 100,0 g |
| Le pH spontané est d'environ 5,45 | |

La composition est maintenue au contact des cheveux pendant 15 minutes, suivi d'un rinçage à l'eau courante et d'un essorage.

On applique ensuite sur la mèche 5 g de la composition (B) telle que définie dans l'exemple 1.

Cette composition est maintenue au contact des cheveux pendant 9 minutes. On rince ensuite la mèche. On lave avec un shampooing à 5% de laurylsulfate de sodium. On obtient de cette façon une mèche dont la couleur est châtain à reflets jaunâtres.

## EXEMPLE 5

On imprègne une mèche de 1 g de cheveux naturels gris à 90% de blancs par 5 g d'une composition (A) suivante :

| Composition (A) | |
|---|---|
| - 2,3-diméthyl 5,6-dihydroxyindole<br>- Ethanol<br>- Eau | 2,5 g<br>40,0 g<br>qsp 100,0 g |
| Le pH spontané est d'environ 5,5 | |

La composition est maintenue au contact des cheveux pendant 15 minutes, suivi d'un rinçage à l'eau courante et d'un essorage. On applique ensuite sur la mèche 5 g de la composition (B) telle que définie dans l'exemple 1.

Cette composition est maintenue au contact des cheveux pendant 10 minutes. On rince ensuite la mèche. On lave avec un shampooing à 5% de laurylsulfate de sodium. On obtient de cette façon une mèche dont la couleur est jaune orangé.

## EXEMPLE 6

On imprègne une mèche de 1 g de cheveux naturels gris à 90% de blancs par 5 g d'une composition (A) suivante :

| Composition (A) | |
|---|---|
| - 5-hydroxy 6-acétoxyindole<br>- 5-acétoxy 6-hydroxyindole<br>- Ethanol<br>- Eau | 0,85 g<br>0,15 g<br>40,0 g<br>qsp 100,0 g |
| Le pH spontané est d'environ 5,55 | |

La composition est maintenue au contact des cheveux pendant 15 minutes, suivi d'un rinçage à l'eau courante et d'un essorage.

On applique ensuite sur la mèche 5 g de la composition (B) telle que définie dans l'exemple 1.

Cette composition est maintenue au contact des cheveux pendant 9 minutes. On rince ensuite la mèche. On lave avec un shampooing à 5% de laurylsulfate de sodium. On obtient de cette façon une mèche dont la couleur est orangé.

## EXEMPLE 7

On imprègne une mèche de 1 g de cheveux naturels gris à 90% de blancs par 5 g d'une composition (A) suivante :

| Composition (A) | |
|---|---|
| - 5-hydroxy 6-méthoxyindole | 2,5 g |
| - Ethanol | 40,0 g |
| - Eau | qsp 100,0 g |
| Le pH spontané est d'environ 5,5 | |

La composition est maintenue au contact des cheveux pendant 15 minutes, suivi d'un rinçage à l'eau courante et d'un essorage.

On applique ensuite sur la mèche 5 g de la composition (B) suivante :

| Composition (B) | |
|---|---|
| - Ethanol | 20,0 g |
| - Nitrite d'amyle | 20,0 g |
| - Acide polyacrylamidométhyl propanesulfonique (sel à 20%) | 25,0 g |
| - Acide chlorhydrique qs pH = 3 | |
| - Eau | qsp 100,0 g |

On obtient une mèche dont la couleur est châtain clair.

## EXEMPLE 8

On prépare les compositions suivantes :

| COMPOSITION COLORANTE : | |
|---|---|
| - N-méthyl 5,6-dihydroxyindole | 2,5 g |
| - Nitrite de sodium | 2,5 g |
| - Dihydrogénophosphate de potassium | 1,3 g |
| - Monohydrogénophosphate de potassium | 3,6 g |
| - Ethanol | 30,0 g |
| - Eau | qsp 100,0 g |
| - pH spontané = 7,4 | |

| COMPOSITION AQUEUSE ACIDE : | |
|---|---|
| - Citrate de sodium, dihydrate | 2,9 g |
| - Acide chlorhydrique qs pH = 3 | |
| - Eau | qsp 100,0 g |

La composition colorante est appliquée pendant 30 minutes sur des cheveux à 90% de blancs. Après rinçage, on applique pendant 9 minutes la composition acide, avant de rincer à nouveau. Après séchage, on obtient des cheveux teints dans une nuance grise uniforme.

**Revendications**

1. Procédé de teinture des fibres kératiniques caractérisé par le fait que l'on applique sur ces fibres au moins une composition (A) contenant, dans un milieu approprié pour la teinture, au moins un dérivé d'indole, répondant à la formule (I) :

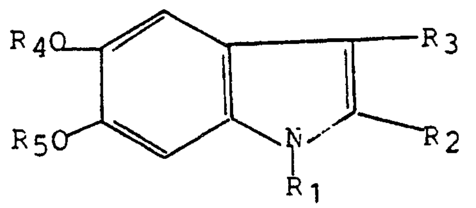

(I)

dans laquelle $R_1$ représente un atome d'hydrogène, un groupement alkyle inférieur ou un groupement $-SiR_9R_{10}R_{11}$ ;
$R_2$ et $R_3$, identiques ou différents, représentent un atome d'hydrogène ou bien un groupement alkyle inférieur, un groupement carboxyle, un groupement alcoxycarbonyle inférieur ou un groupement $-COOSiR_9R_{10}R_{11}$ ;
$R_4$ et $R_5$, identiques ou différents, représentent un atome d'hydrogène, un groupement alkyle linéaire ou ramifié en $C_1$-$C_{20}$, un groupement formyle, un groupement acyle en $C_2$-$C_{20}$, linéaire ou ramifié, un groupement alcénoyle en $C_3$-$C_{20}$, linéaire ou ramifié, un groupement $-SiR_9R_{10}R_{11}$, un groupement $-P(O)-(OR_6)_2$, un groupement $R_6OSO_2$-, ou bien $R_4$ et $R_5$, conjointement avec les atomes d'oxygène auxquels ils sont rattachés, forment un cycle contenant éventuellement un groupement carbonyle, un groupement méthylène, un groupement thiocarbonyle ou un groupement :
$>P(O)OR_6$, ou bien $>CR_7R_8$ ;
$R_6$ et $R_7$ représentant un atome d'hydrogène ou un groupement alkyle inférieur, $R_8$ représentant un groupement alcoxy inférieur ou un groupement mono ou dialkylamino, $R_9$, $R_{10}$ et $R_{11}$, identiques ou différents, représentant des groupements alkyle inférieurs, linéaires ou ramifiés, l'un au moins des radicaux $R_1$ à $R_5$ étant différent d'hydrogène, et les sels d'addition avec des acides minéraux ou organiques comme par exemple les chlorhydrates et bromhydrates ainsi que les sels de métaux alcalins, alcalino-terreux ou d'amines correspondants,
l'application de la composition (A) étant suivie par l'application d'une composition (B), constituée par une composition aqueuse présentant un pH acide, la composition (A) ou (B) contenant au moins un nitrite.

2. Procédé de teinture selon la revendication 1, caractérisé par le fait que les dérivés d'indole sont choisis parmi les composés de formule :

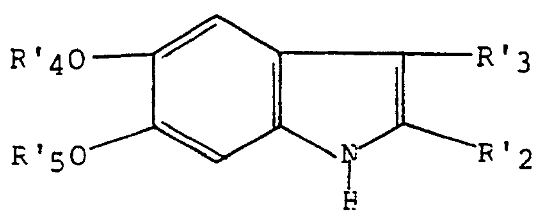

(II)

dans laquelle $R'_2$ et $R'_3$, identiques ou différents, représentent un atome d'hydrogène, un groupement alkyle inférieur en $C_1$-$C_6$, un groupement carboxyle, un groupement alcoxycarbonyle inférieur en $C_1$-$C_6$ pour le groupement alcoxy, $R'_4$ et $R'_5$, identiques ou différents, représentent un atome d'hydrogène, un groupement alkyle inférieur en $C_1$-$C_6$, un groupement acyle linéaire en $C_2$-$C_{20}$, et de préférence en $C_2$-$C_{14}$, un groupement triméthylsilyle, ou bien $R'_4$ et $R'_5$ conjointement avec les atomes d'oxygène auxquels ils sont rattachés, forment un cycle méthylènedioxy ou carbonyldioxy, l'un au moins des radicaux $R'_4$ ou $R'_5$ étant différent d'hydrogène.

3. Procédé selon l'une quelconque des revendications 1 ou 2, caractérisé par le fait que les dérivés d'indole sont choisis parmi le 5-méthoxy 6-hydroxyindole, le 1-méthyl, 5,6-dihydroxyindole, le 3-méthyl 5,6-dihydroxyindole, le 2-méthyl 5,6-dihydroxyindole, le 2,3-diméthyl 5,6-dihydroxyindole, le 5,6-di-(triméthylsilyloxy)indole, le 5-(ou 6-)myristoyloxy 6-(ou 5-)hydroxyindole, le 5,6-(méthylènedioxy)indole, le 5-acétoxy 6-hydroxy indole, le 5-hydroxy 6-acétoxyindole, le 2-éthoxycarbonyl 5,6-dihydroxyindole, le 5-hydroxy 6-méthoxyindole, le 5,6-carbonyldioxyindole et le 2-carboxy 5,6-dihydroxyindole et leurs sels.

4. Procédé de teinture selon l'une quelconque des revendications 1 à 3, caractérisé par le fait que le nitrite est choisi parmi les nitrites de métaux alcalins, alcalino-terreux ou d'ammonium ou d'un autre cation

cosmétiquement acceptable, ainsi que des dérivés organiques de nitrite ou des vecteurs de nitrite générant par transformation un nitrite.

5. Procédé selon l'une quelconque des revendications 1 à 4, caractérisé par le fait que le nitrite est choisi parmi les nitrites de sodium, de potassium ou d'ammonium.

6. Procédé selon l'une quelconque des revendications 1 à 5, caractérisé par le fait que les dérivés d'indole tels que définis dans l'une quelconque des revendications 1 à 3, sont utilisés dans des concentrations comprises entre 0,01 et 0,3 mole/litre.

7. Procédé selon l'une quelconque des revendications 1 à 6, caractérisé par le fait que le nitrite est utilisé dans des proportions comprises entre 0,02 et 1 mole/litre.

8. Procédé selon l'une quelconque des revendications 1 à 7, caractérisé par le fait que le pH de la composition (A) est compris entre 2 et 10.

9. Procédé selon l'une quelconque des revendications 1 à 8, caractérisé par le fait que le pH de la composition (B) est compris entre 2 et 6.

10. Procédé selon l'une quelconque des revendications 1 à 9, caractérisé par le fait que les compositions (A) ou (B) contiennent en plus de l'eau, au moins un ou plusieurs solvants organiques cosmétiquement acceptables.

11. Procédé selon les revendications 1 à 10, caractérisé par le fait qu'au moins l'une des compositions (A) ou (B) contient en plus un colorant direct, un colorant d'oxydation, un coupleur ou un colorant d'oxydation rapide.

12. Procédé selon l'une quelconque des revendications 1 à 11, caractérisé par le fait qu'au moins une des compositions (A) ou (B) contient en plus du colorant dérivé d'indole tel que défini dans l'une quelconque des revendications 1 à 3, le 5,6-dihydroxy indole.

13. Procédé de teinture des fibres kératiniques selon l'une quelconque des revendications 1 à 12, caractérisé par le fait que l'on applique dans un premier temps, une composition (A) contenant dans un milieu approprié pour la teinture les dérivés d'indole tels que définis dans l'une quelconque des revendications 1 à 3 et dans un second temps, un composition (B) contenant dans un milieu aqueux ajusté à pH acide, un nitrite.

14. Procédé selon l'une quelconque des revendications 1 à 12, caractérisé par le fait que l'on applique dans un premier temps une composition (A′) contenant dans un milieu approprié pour la teinture et présentant un pH neutre ou alcalin, un dérivé d'indole tel que défini dans l'une quelconque des revendications 1 à 3 et un nitrite, et dans un second temps, on applique une composition aqueuse ajustée à un pH acide.

15. Procédé selon l'une quelconque des revendications 1 à 14, caractérisé par le fait que l'on fait suivre la première étape par une étape de rinçage.

16. Procédé selon l'une quelconque des revendications 1 à 15, caractérisé par le fait que la composition appliquée dans le premier temps est maintenue au contact des cheveux pendant une durée de 5 à 30 minutes et que la composition appliquée dans le second temps est maintenue au contact des cheveux pendant une durée également de 5 à 30 minutes.

17. Application du procédé tel que défini dans l'une quelconque des revendications 1 à 16 à la teinture des cheveux humains.

18. Composition destinée à être utilisée pour la teinture des fibres kératiniques et en particulier des cheveux humains, caractérisée par le fait qu'elle contient dans un milieu aqueux à pH neutre ou alcalin, approprié pour la teinture, au moins un dérivé d'indole tel que défini dans l'une quelconque des revendications 1 à 3 et au moins un nitrite.

19. Dispositif à plusieurs compartiments ou "kit" destiné à être utilisé dans la teinture des fibres kératiniques et en particulier des cheveux humains, caractérisé par le fait qu'il comporte, dans un premier compartiment, une composition (A) contenant dans un milieu approprié pour la teinture, un dérivé d'indole tel que défini dans l'une quelconque des revendications 1 à 3, et dans un compartiment (B) une composition aqueuse présentant un pH acide contenant un nitrite.

20. Dispositif à plusieurs compartiments ou "kit" destiné à être utilisé dans la teinture des fibres kératiniques et en particulier des cheveux humains, caractérisé par le fait qu'il comporte dans un premier compartiment une composition (A′) telle que définie dans la revendication 18, et dans un second compartiment, une composition aqueuse acide.

Revendications pour l'Etat contractant suivant: ES

1. Procédé de teinture des fibres kératiniques, caractérisé par le fait que l'on applique sur ces fibres au moins une composition (A) contenant, dans un milieu approprié pour la teinture, au moins un dérivé

d'indole, répondant à la formule (I) :

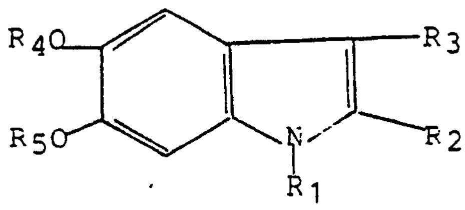

(I)

dans laquelle $R_1$ représente un atome d'hydrogène, un groupement alkyle inférieur ou un groupement $-SiR_9R_{10}R_{11}$ ;
$R_2$ et $R_3$, identiques ou différents, représentent un atome d'hydrogène ou bien un groupement alkyle inférieur, un groupement carboxyle, un groupement alcoxycarbonyle inférieur ou un groupement $-COOSiR_9R_{10}R_{11}$ ;
$R_4$ et $R_5$, identiques ou différents, représentent un atome d'hydrogène, un groupement alkyle linéaire ou ramifié en $C_1$-$C_{20}$, un groupement formyle, un groupement acyle en $C_2$-$C_{20}$, linéaire ou ramifié, un groupement alcénoyle en $C_3$-$C_{20}$, linéaire ou ramifié, un groupement $-SiR_9R_{10}R_{11}$, un groupement $-P(O)(OR_6)_2$, un groupement $R_6OSO_2$-, ou bien $R_4$ et $R_5$, conjointement avec les atomes d'oxygène auxquels ils sont rattachés, forment un cycle contenant éventuellement un groupement carbonyle, un groupement méthylène, un groupement thiocarbonyle ou un groupement :
$>P(O)OR_6$, ou bien $>CR_7R_8$ ;
$R_6$ et $R_7$ représentant un atome d'hydrogène ou un groupement alkyle inférieur, $R_8$ représentant un groupement alcoxy inférieur ou un groupement mono ou dialkylamino, $R_9$, $R_{10}$ et $R_{11}$, identiques ou différents, représentant des groupements alkyle inférieurs, linéaires ou ramifiés, l'un au moins des radicaux $R_1$ à $R_5$ étant différent d'hydrogène, et les sels d'addition avec des acides minéraux ou organiques comme par exemple les chlorhydrates et bromhydrates ainsi que les sels de métaux alcalins, alcalino-terreux ou d'amines correspondants,
l'application de la composition (A) étant suivie par l'application d'une composition (B), constituée par une composition aqueuse présentant un pH acide, la composition (A) ou (B) contenant au moins un nitrite.

2. Procédé de teinture selon la revendication 1, caractérisé par le fait que les dérivés d'indole sont choisis parmi les composés de formule :

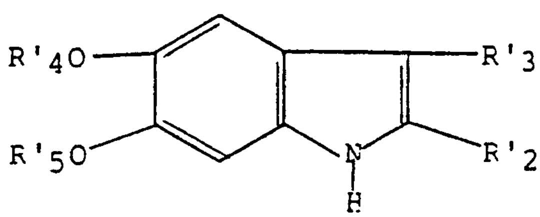

(II)

dans laquelle $R'_2$ et $R'_3$, identiques ou différents, représentent un atome d'hydrogène, un groupement alkyle inférieur en $C_1$-$C_6$, un groupement carboxyle, un groupement alcoxycarbonyle inférieur en $C_1$-$C_6$ pour le groupement alcoxy, $R'_4$ et $R'_5$, identiques ou différents, représentent un atome d'hydrogène, un groupement alkyle inférieur en $C_1$-$C_6$, un groupement acyle linéaire en $C_2$-$C_{20}$, et de préférence en $C_2$-$C_{14}$, un groupement triméthylsilyle, ou bien $R'_4$ et $R'_5$ conjointement avec les atomes d'oxygène auxquels ils sont rattachés, forment un cycle méthylènedioxy ou carbonyldioxy, l'un au moins des radicaux $R'_4$ ou $R'_5$ étant différent d'hydrogène.

3. Procédé selon l'une quelconque des revendications 1 ou 2, caractérisé par le fait que les dérivés d'indole sont choisis parmi le 5-méthoxy 6-hydroxyindole, le 1-méthyl, 5,6-dihydroxyindole, le 3-méthyl 5,6-dihydroxyindole, le 2-méthyl 5,6-dihydroxyindole, le 2,3-diméthyl 5,6-dihydroxyindole, le 5,6-di-(triméthylsilyloxy)indole, le 5-(ou 6-)myristoyloxy 6-(ou 5-)hydroxyindole, le 5,6-(méthylènedioxy)indole, le 5-acétoxy 6-hydroxy indole, le 5-hydroxy 6-acétoxyindole, le 2-éthoxycarbonyl 5,6-dihydroxyindole, le 5-hydroxy 6-méthoxyindole, le 5,6-carbonyldioxyindole et le 2-carboxy 5,6-dihydroxyindole et leurs sels.

4. Procédé de teinture selon l'une quelconque des revendications 1 à 3, caractérisé par le fait que le nitrite est choisi parmi les nitrites de métaux alcalins, alcalino-terreux ou d'ammonium ou d'un autre cation cosmétiquement acceptable, ainsi que des dérivés organiques de nitrite ou des vecteurs de nitrite générant par transformation un nitrite.

5. Procédé selon l'une quelconque des revendications 1 à 4, caractérisé par le fait que le nitrite est choisi parmi les nitrites de sodium, de potassium ou d'ammonium.

6. Procédé selon l'une quelconque des revendications 1 à 5, caractérisé par le fait que les dérivés d'indole tels que définis dans l'une quelconque des revendications 1 à 3, sont utilisés dans des concentrations comprises entre 0,01 et 0,3 mole/litre.

7. Procédé selon l'une quelconque des revendications 1 à 6, caractérisé par le fait que le nitrite est utilisé dans des proportions comprises entre 0,02 et 1 mole/litre.

8. Procédé selon l'une quelconque des revendications 1 à 7, caractérisé par le fait que le pH de la composition (A) est compris entre 2 et 10.

9. Procédé selon l'une quelconque des revendications 1 à 8, caractérisé par le fait que le pH de la composition (B) est compris entre 2 et 6.

10. Procédé selon l'une quelconque des revendications 1 à 9, caractérisé par le fait que les compositions (A) ou (B) contiennent en plus de l'eau, au moins un ou plusieurs solvants organiques cosmétiquement acceptables.

11. Procédé selon les revendications 1 à 10, caractérisé par le fait qu'au moins l'une des compositions (A) ou (B) contient en plus un colorant direct, un colorant d'oxydation, un coupleur ou un colorant d'oxydation rapide.

12. Procédé selon l'une quelconque des revendications 1 à 11, caractérisé par le fait qu'au moins une des compositions (A) ou (B) contient en plus du colorant dérivé d'indole tel que défini dans l'une quelconque des revendications 1 à 3, le 5,6-dihydroxy indole.

13. Procédé de teinture des fibres kératiniques selon l'une quelconque des revendications 1 à 12, caractérisé par le fait que l'on applique dans un premier temps, une composition (A) contenant dans un milieu approprié pour la teinture les dérivés d'indole tels que définis dans l'une quelconque des revendications 1 à 3 et dans un second temps, un composition (B) contenant dans un milieu aqueux ajusté à pH acide, un nitrite.

14. Procédé selon l'une quelconque des revendications 1 à 12, caractérisé par le fait que l'on applique dans un premier temps une composition (A′) contenant dans un milieu approprié pour la teinture et présentant un pH neutre ou alcalin, un dérivé d'indole tel que défini dans l'une quelconque des revendications 1 à 3 et un nitrite, et dans un second temps, on applique une composition aqueuse ajustée à un pH acide.

15. Procédé selon l'une quelconque des revendications 1 à 14, caractérisé par le fait que l'on fait suivre la première étape par une étape de rinçage.

16. Procédé selon l'une quelconque des revendications 1 à 15, caractérisé par le fait que la composition appliquée dans le premier temps est maintenue au contact des cheveux pendant une durée de 5 à 30 minutes et que la composition appliquée dans le second temps est maintenue au contact des cheveux pendant une durée également de 5 à 30 minutes.

17. Composition destinée à être utilisée pour la teinture des fibres kératiniques et en particulier des cheveux humains, caractérisée par le fait qu'elle contient dans un milieu aqueux à pH neutre ou alcalin, approprié pour la teinture, au moins un dérivé d'indole tel que défini dans l'une quelconque des revendications 1 à 3 et au moins un nitrite.

18. Procédé de préparation d'une composition de teinture des fibres kératiniques, caractérisé par le fait que l'on introduit, dans un milieu approprié pour la teinture, au moins un dérivé d'indole répondant à la formule (I) :

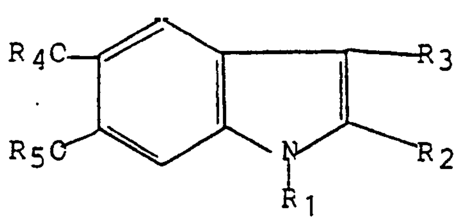

(I)

dans laquelle $R_1$ représente un atome d'hydrogène, un groupement alkyle inférieur ou un groupement $-SiR_9R_{10}R_{11}$ ;

$R_2$ et $R_3$, identiques ou différents, représentent un atome d'hydrogène ou bien un groupement alkyle inférieur, un groupement carboxyle, un groupement alcoxycarbonyle inférieur ou un groupement $-COOSiR_9R_{10}R_{11}$ ;

$R_4$ et $R_5$, identiques ou différents, représentent un atome d'hydrogène, un groupement alkyle linéaire ou ramifié en $C_1$-$C_{20}$, un groupement formyle, un groupement acyle en $C_2$-$C_{20}$, linéaire ou ramifié, un

groupement alcénoyle en $C_3$-$C_{20}$, linéaire ou ramifié, un groupement $-SiR_9R_{10}R_{11}$, un groupement $-P(O)(OR_6)_2$, un groupement $R_6OSO_2-$, ou bien $R_4$ et $R_5$, conjointement avec les atomes d'oxygène auxquels ils sont rattachés, forment un cycle contenant éventuellement un groupement carbonyle, un groupement méthylène, un groupement thiocarbonyle ou un groupement :

$>P(O)OR_6$, ou bien $>CR_7R_8$ ;

$R_6$ et $R_7$ représentant un atome d'hydrogène ou un groupement alkyle inférieur, $R_8$ représentant un groupement alcoxy inférieur ou un groupement mono ou dialkylamino, $R_9$, $R_{10}$ et $R_{11}$, identiques ou différents, représentant des groupements alkyle inférieurs, linéaires ou ramifiés, l'un au moins des radicaux $R_1$ à $R_5$ étant différent d'hydrogène, et les sels d'addition avec des acides minéraux ou organiques comme par exemple les chlorhydrates et bromhydrates ainsi que les sels de métaux alcalins, alcalino-terreux ou d'amines correspondants, dans des proportions de 0,01 à 0,3 moles/litre et au moins un nitrite dans des proportions de 0,02 à 0,1 mole/litre.

19. Procédé de préparation d'un agent de teinture des fibres kératiniques à plusieurs composants, caractérisé par le fait que l'on prépare un premier composant en introduisant dans un milieu approprié pour la teinture, un dérivé d'indole tel que défini dans l'une quelconque des revendications 1 à 3 et que l'on prépare un second composant en introduisant dans un milieu aqueux à pH acide, un nitrite.

20. Dispositif à plusieurs compartiments ou "kit" destiné à être utilisé dans la teinture des fibres kératiniques et en particulier des cheveux humains, caractérisé par le fait qu'il comporte, dans un premier compartiment, une composition (A) contenant dans un milieu approprié pour la teinture, un dérivé d'indole tel que défini dans l'une quelconque des revendications 1 à 3, et dans un compartiment (B) une composition aqueuse présentant un pH acide contenant un nitrite.

21. Dispositif à plusieurs compartiments ou "kit" destiné à être utilisé dans la teinture des fibres kératiniques et en particulier des cheveux humains, caractérisé par le fait qu'il comporte dans un premier compartiment une composition (A′) telle que définie dans la revendication 17, et dans un second compartiment, une composition aqueuse acide.

Office européen des brevets

# RAPPORT DE RECHERCHE EUROPEENNE

Numero de la demande

EP 89 40 3489

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,P | FR-A-2 626 173 (L'OREAL)<br>* En entier * | 1-21 | A 61 K 7/13 |
| A | GB-A-2 187 456 (L'OREAL)<br>* En entier * | 1-3,6,8 -20 | |
| A | EP-A-0 271 186 (REPLIGEN CORP.)<br>* Revendications * | 1-18 | |
| A | HOUBEN-WEYL: "Methoden der organischen Chemie", vol. VII/3b, Chinone, partie II, 1979, pages 30-35, G. Thieme-Verlag, Stuttgart, DE<br>* En entier * | 1-5 | |
| A | CHEMICAL ABSTRACTS, vol. 89, 1978, page 200, résumé no. 2111y, Columbus, Ohio, US; F.N. BOCTOR et al.: "A colorimetric method for the determination of indole, and its application to assay of tryptophanase", & ANAL. BIOCHEM. 1978, 86(2), 457-62 | 1-5 | DOMAINES TECHNIQUES RECHERCHES (Int. Cl.5)<br>A 61 K |

Le présent rapport a été établi pour toutes les revendications

| Lieu de la recherche | Date d'achèvement de la recherche | Examinateur |
|---|---|---|
| LA HAYE | 24-04-1990 | FISCHER J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant